Europäisches Patentamt

**European Patent Office**

**Office européen des brevets**

(19)

(11) Publication number: **0 135 390**
**A2**

(12) # EUROPEAN PATENT APPLICATION

(21) Application number: **84306235.7**

(22) Date of filing: **12.09.84**

(51) Int. Cl.⁴: **A 61 M 11/00**
**A 61 M 15/00**

(30) Priority: **19.09.83 GB 8325047**

(43) Date of publication of application:
**27.03.85 Bulletin 85/13**

(84) Designated Contracting States:
**DE FR GB SE**

(71) Applicant: **House, David Howard**
**Acorns Lock Lane**
**Birdham Chichester W. Sussex(GB)**

(72) Inventor: **House, David Howard**
**Acorns Lock Lane**
**Birdham Chichester W. Sussex(GB)**

(74) Representative: **Arthur, Bryan Edward et al,**
**Withers & Rogers 4 Dyer's Buildings Holborn**
**London EC1N 2JT(GB)**

(54) **Improvements in and relating to nebulizers.**

(57) This invention relates to a screening unit suitable for use in conjunction with a nebulizer. A nebulizer is a device for producing an aerosol spray of liquid particles entrained in a fluid stream, for example air or a gas. An aerosol spray produced by a nebulizer is a convenient way of administering drugs and other medicines by inhalation for the treatment of respiratory and other internal disorders in humans.

In more detail, the invention relates to a screening unit for use in conjunction with a nebulizer, the screening unit comprising a body, an inlet for receiving liquid particles entrained in a gas stream, a screen comprising a multiplicity of tortuous paths for removing from the stream particles greater than a predetermined size, a baffle disposed upstream of the screen and in the flow path of the gas stream, the baffle including at least one baffle member for directing the incoming gas stream in a contraflow pattern relative to the incoming gas stream to effect primary filtering in the screen, and an outlet for the so-filtered gas stream.

EP 0 135 390 A2

./...

Croydon Printing Company Ltd

FIGURE 1.

# IMPROVEMENTS IN AND RELATING TO NEBULIZERS

This invention relates to a screening unit suitable for use in conjunction with a nebulizer. A nebulizer is a device for producing an aerosol spray of liquid particles entrained in a fluid stream, for example air or a gas. An aerosol spray produced by a nebulizer is a convenient way of administering drugs and other medicines by inhalation for the treatment of respiratory and other internal disorders in humans.

Known nebulizer devices produce aerosol sprays containing a range of particle sizes. If the particles are too small there is a tendency for them to be exhaled by a patient. On the other hand if the particles are too large there is a tendency for them to be deposited within the upper regions of the respiratory system of a patient and not, for example, to reach a lung or other part of a human body where treatment is required.

Two forms of nebulizer are described and claimed in U.S. Patent Nos. 3,762,409 and 4,333,450, issued to Victor E. Lester. Briefly, the nebulizer of U.S. Patent 3,762,409 includes an enclosed container having an aerosol outlet, a nozzle assembly extending into the container, and a diffuser-baffle assembly in the container.

The nozzle assembly includes a gas nozzle and a spray nozzle with spaced and coaxially positioned orifices. The diffuser-baffle assembly is located in the spray path of the spray orifice and includes a nose portion which extends towards the orifices and is coaxially positioned therewith.

Briefly, the nebulizer of U.S. Patent 4,333,450 includes a manifold body and a nebulizer unit having a nozzle assembly. The mainfold body includes a main supply inlet, an exhalation outlet, a patient connector port and a diaphragm assembly for controlling the exhalation port.

It is an object of the present invention to provide a nebulizer and a screening unit therefor which may be a throw-away or a rechargeable unit including means for regulating the particle size of liquid within prescribed limits.

According to one aspect of the present invention, there is provided a screening unit, for use in conjunction with a nebulizer, the unit comprising a body including an inlet adapted to receive liquid particles entrained in a gas stream, a screen comprising a multiplicity of tortuous paths and means for directing the so-entrained liquid particles through the screen which preferentially filters from the stream particles greater than a predetermined size.

The invention also includes a screening unit comprising a body, an inlet for receiving liquid particles entrained in a gas stream, a screen comprising a multiplicity of tortuous paths for removing from the stream particles greater than a predetermined size, a baffle disposed upstream of the screen and in the flow path of the gas stream, the baffle including at least one baffle member for directing the incoming gas stream in a contraflow pattern relative to the incoming gas stream to effect primary filtering of the incoming gas stream prior to secondary filtering in the screen, and an outlet for the so-filtered gas stream.

Preferably, each baffle member is tubular having an open end for receiving the incoming gas stream and a closed end for directing the gas stream in the contraflow pattern.

The screening unit may include a plurality of tubular baffles of different diameter and disposed around a common axis and the axial extent of at least one tubular baffle is different from the remaining tubular baffles.

The tubular baffles disposed around a common axis direct the incoming gas stream in a contraflow pattern including a plurality of U-turns thereby increasing primary filtration.

The multiplicity of tortuous paths may be formed using a plurality of superimposed gauzes made from a material which is inert relative to the liquid particles and in a gas stream.

According to one form of the present invention, the screen may comprise a plurality of bodies of regular or irregular geometric shape assembled so that interstitial spaces between adjacent bodies form the multiplicity of tortuous paths. The said bodies may be of rounded form such as spheroids, solids of revolution having rounded ends, ellipsoids and the like. Preferably, the bodies are spherical and made from a material which is inert relative to the liquid particles and the gas stream, for example glass, stainless steel or ceramic. If desired, the bodies may be made from materials which are not inert relative to the liquid particles and the gas stream but, in such instances, the bodies are coated with an inert material such as glass or a ceramic.

The glass-spheres may be assembled in layers supported on gauzes made from, for example, stainless steel wire. Further we have found that it is convenient in certain circumstances to divide the screen into two or more subsidiary screens with a space therebetween. In use, the first subsidiary screen, when considered in the direction of flow of the particles, collects the relatively large and unwanted particles whereas the second and subsequent subsidiary screens remove particles of gradually diminishing size until substantially only those particles remaining in the stream are those required for inhalation by a patient.

In a preferred form of screen, glass-spheres are disposed between two surfaces (herein referred to as an inner and outer surface, respectively) which define a generally divergent or convergent path relative to the direction of the incoming particle laden gas stream.

The said two surfaces are, preferably, coaxial substantially parallel when viewed in cross-section and at least partially conical with the apex (imaginary or real) directed towards or away from the direction of the incoming particle laden gas stream. The separated or filtered liquid particles naturally coalesce on the said outer surface and drain back into a liquid reservoir from which the particles were originally produced. Thus, the separated or filtered particles do not serve as mobile cites attracting and collecting additional liquid particles of such a size which would, otherwise, normally pass through the screen.

The present invention also includes a nebulizer fitted with a screening unit as described above, the nebulizer also including a nozzle assembly comprising a gas nozzle and a spray nozzle for producing an aerosol spray which is directed towards the baffle system for primary filtration prior to secondary filtration in the screen.

Various embodiments of screening unit in accordance with the present invention will now be presented by way of example, with reference to the accompanying drawings, in which:

Figure 1 is a diagrammatic section through a nebulizer fitted with one form of planar screening unit according to the invention;

Figure 2 is a modification of the embodiment of Figure 1 showing a part-conical and convergent screening unit;

Figure 3 is a sectional plan taken on line x-x of Figure 2;

Figure 4 is a further modification of the embodiment of Figure 1 showing a part-conical and divergent screening unit;

Figure 5 is a section taken through a divergent/convergent screening unit, and

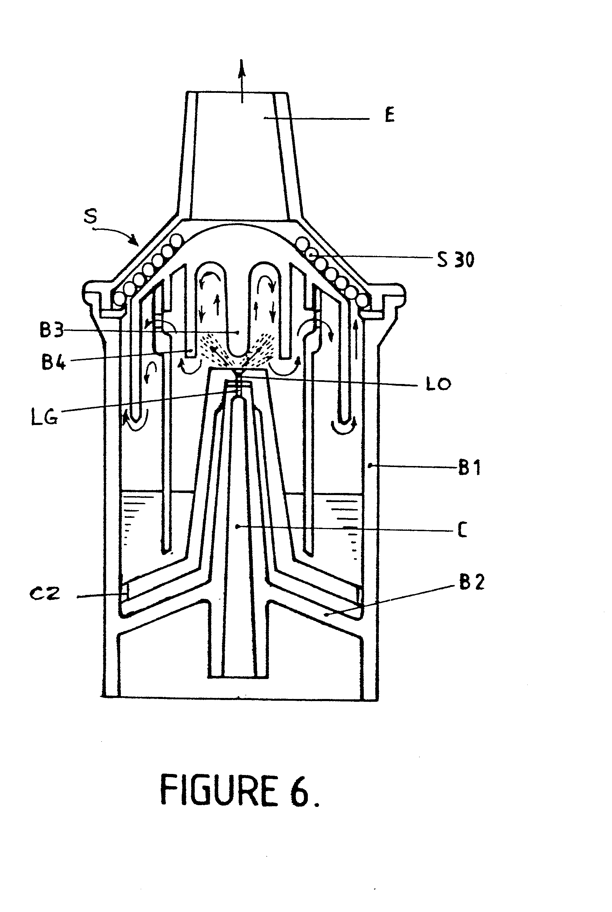

Figure 6 is a modification of the embodiment shown in Figure 2.

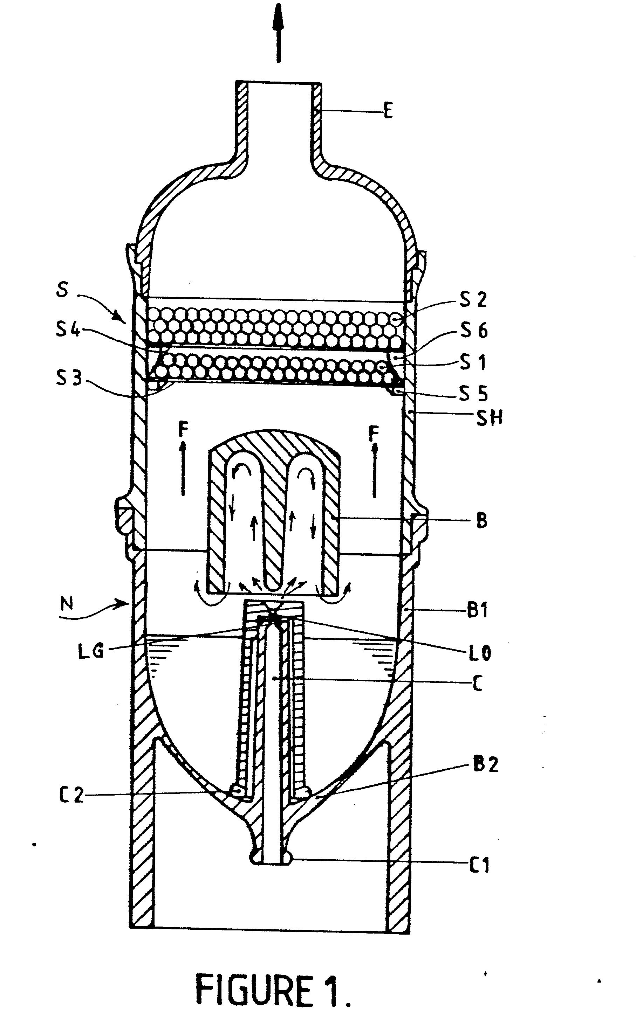

Figure 1 shows a screening unit S divided into two subsidiary screens S1 and S2 comprising two and three layers of glass spheres respectively with the spheres of each subsidiary screen supported by stainless steel gauzes S3 and S4. As shown in Figure 1 the lower gauze S3 is supported on a flange S5 and the upper gauze is supported on an annular spacer S6 which rests on the gauze S3. In order to retain the glass spheres in layers, as shown, an additional layer of gauze (not shown) may be located above the uppermost layers of glass spheres of each of the subsidiary screens S1 and S2. As shown, the screening unit S is mounted above a baffle system B and aerosol spray forming nozzle system C of a nebulizer N having a body B1 formed with a funnel base B2. The funnel base B2 and body B1 together form a container for a reservoir of liquid to be converted into the aerosol. As shown in Figure 1, liquid from the reservoir enters the nozzle system through a plurality of passages of which C2 is shown. The baffle B may be supported by means of arms extending radially between the baffle

0135390

B and either a housing SH of the screening units or the body B1 of the nebulizer. If desired, however, the baffle B may be suspended from the flange S5 by a plurality of arms (not shown). Further, the nozzle system C includes an extension C1 for receiving a stream of air or a gas under pressure for the purpose of producing the aerosol which flows in direction F, through the screen unit S and exits at outlet E.

The baffle system B includes an impaction baffle B3 disposed immediately above a liquid orifice LO which in turn is disposed above an air or gas nozzle LG. The baffle system also includes a tubular screening baffle B4 which serves to produce a contraflow pattern in the liquid particle laden gas stream. As shown, the contraflow pattern results in the gas stream following a number of U-turns before entering the screening unit S.

A conventional manifold (not shown) is, in practice, fitted to the outlet E for connecting the unit to patient.

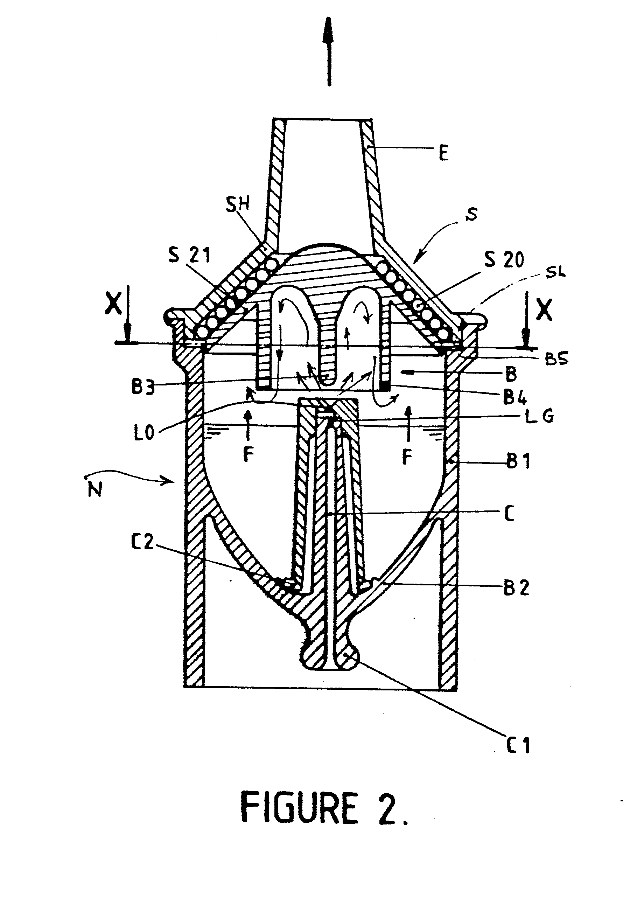

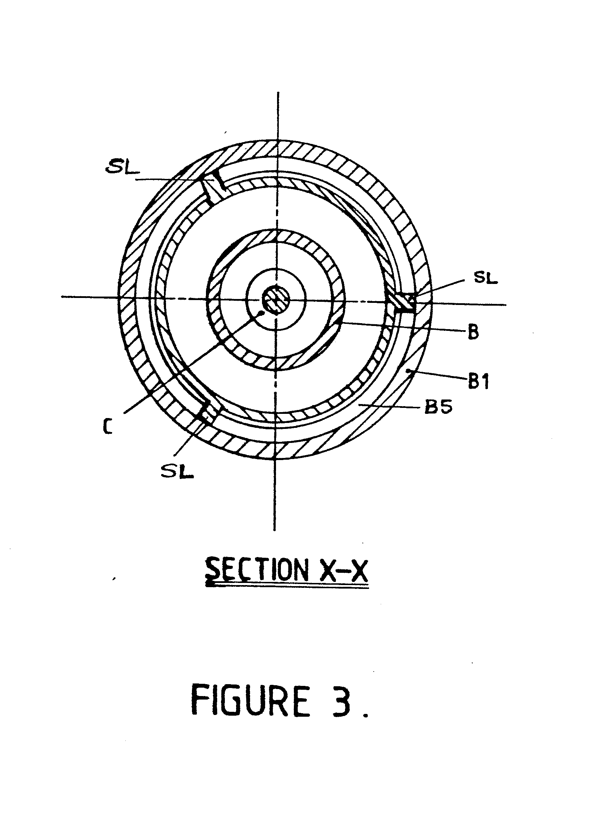

Figures 2 and 3 show a modification of the embodiment of Figure 1 and like parts are represented by the same reference numbers. Figure 2 shows a different screen unit including a convergent frusto-conical array of glass spheres
S20. Although in Figure 2, the screen S20
converges in the direction of flow F of the aerosol
and shows a single layer of glass spheres, a
plurality of layers may be used. In the
modification of Figures 2 and 3, the baffle B
is moulded integrally with component S21 of the
screen unit. In use, liquid particles greater
than a predetermined size coalesce and run down
the upper conical (i.e., outer) surface of the
screen housing SH and drain into the reservoir
formed in the nebulizer body Bl. As previously
mentioned, liquid from the reservoir is drawn
into the nozzle system C through passages C2
disposed around the foot of the nozzle system
C. As shown in Figure 3, the screen unit S is
supported on the housing Bl by means of three
lugs SL which rest or are located in notches (not
shown) formed in shoulder B5 of the housing Bl.

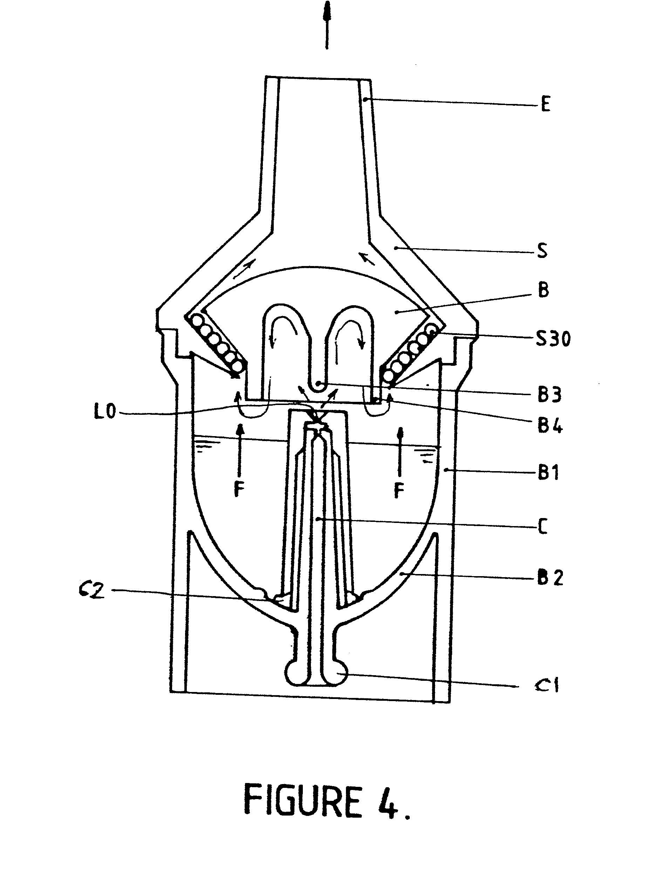

Figure 4 shows a modification of Figures
2 and 3 in which the screen consists of a frusto-
conical array of glass spheres S30 which is
divergent relative to the direction of flow F.
As with the embodiment of Figures 2 and 3, if
desired, a plurality of layers of glass spheres
may be used.

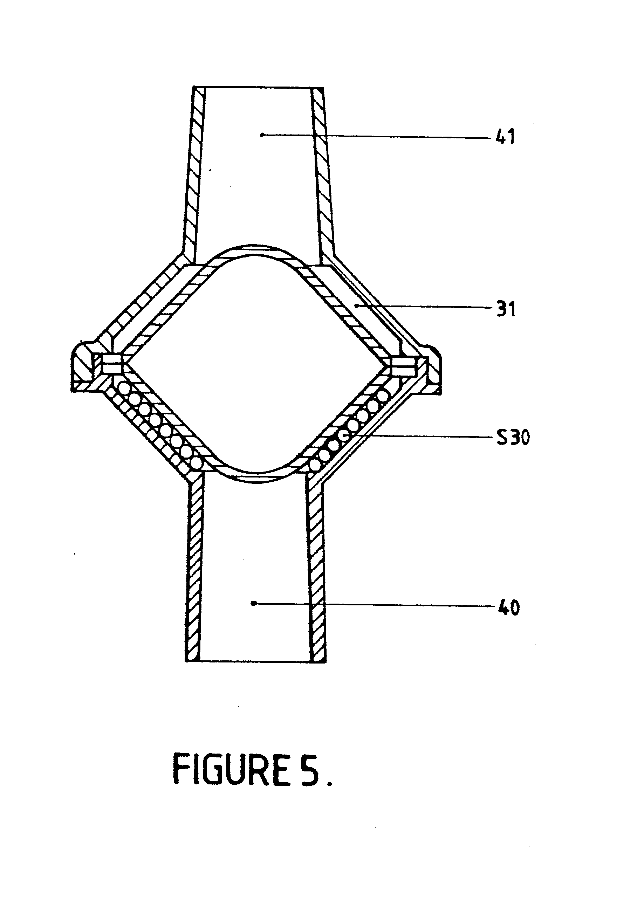

A similar arrangement of the embodiment of Figure 4 is shown in Figure 5 which shows an individual screening unit with an inlet 40 and an outlet 41 for connection with a nebulizer and patient manifold respectively. In the embodiment of Figure 5 the divergent frusto-conical array of glass spheres S30 is followed by a frusto-conical passage S31 which leads to the outlet 41. The frusto-conical passage 31 may also contain one or more layers of glass spheres.

The construction of the nebulizer according to the invention, the choice of air or gas orifice LG (Figure 1), the size of liquid orifice L0 (Figure 1), size impaction baffle B3, and of tubular screening baffle B4, all play a part in determining a consistent size of aerosol particle produced. It is also of importance to know the exact distribution of these particles. Desirably what is required is a nebulizer which will produce the majority of its output in particles less than 5 microns mass median diameter and with as large a number as possible of the particles having a mass median diameter below 1 micron.

Referring again to Figure 1, after production of the aerosol, the gas laden with liquid particles is free to rise from the nebulizer chamber into a chamber where it meets the screen S1 supporting a layer of spheres of a know size. As the aerosol passes through the interstitial space between these spheres it follows a number of known paths containing a known number of turns and larger particles are deposited on the screen. These particles eventually condense or coalesce and fall back into the nebulizer chamber; the filtered aerosol is then free to pass further up the centre chamber through the second screen S2 supporting a predetermined and larger number of spheres of a know and smaller size than the spheres of the first screen S1.

Since each aerosol particle has a known mass and a known velocity, it also has a known momentum. As an aerosol particle passes through the interstitial space of this screen of spheres S2 it again must make a known number of turns of a known degree to pass therethrough. It is mathematically calculated that only particles smaller than a particular size can pass through the column of spheres if the spheres are also of a particular size. The aerosol is then free to exit from the unit through a manifold and thence to a patient.

In each of those embodiments of screens

- 12 -

0135390

including one or more convergent and or divergent layers of glass spheres, the spheres are preferably supported in channels defined by fins or webs extending from the walls defining the convergent or divergent screen. The walls of the chamber may be continuous or include breaks disposed at a pitch which may be less than or greater than the diameter of the glass spheres. The existence of such breaks assists in drainage of any condensed or coalesced liquid from the aerosol spray. The channels may follow a spiral and threadlike path along the supporting screen wall. Thus, referring to the embodiment of Figure 2, the convergent frusto-conical surface of component S21 is, preferably, formed with a continuous spiral channel into which the glass spheres may be fed via outlet E. In such a case, when the required numbers of spheres has been fed into the channel a small plug may be inserted in the channel to prevent loss of the spheres during transit and use.

We have found good results are obtained when convergent and divergent chambers converge or diverge with respect to their axes of symmetry within an angular range of 15° to 75° and, preferably, within the range of 25° to 50°. More preferably, the chambers converge or diverge within an angular range of 35° to 45°.

- 13 -

0135390

Further, the glass spheres and/or the baffles may be coated with a material such as silicone so as to assist in repulsion and removal of any liquid which may collect or coalesce on the baffles and/or spheres. We have found that glass spheres having a diameter of 3mm are particularly useful in the screening unit according to the invention. The various components of the nebulizer/screening unit may be moulded from a natural or synthetic plastics material.

- 14 -

0135390

<center>CLAIMS</center>

1. A screening unit comprising a body, an inlet adapted to receive liquid particles entrained in a gas stream, a screen comprising a multiplicity of tortuous paths and means for directing the so-entrained liquid particles through the screen which preferentially filters from the stream particles greater than a predetermined size and an outlet for the so-filtered gas stream.

2. A screen unit comprising a body, an inlet for receiving liquid particles entrained in a gas stream, a screen comprising a multiplicity of tortuous paths for removing from the stream particles greater than a predetermined size, a baffle disposed upstream of the screen and in the flow path of the gas stream, the baffle including at least one baffle member for directing the incoming gas stream in a contraflow pattern relative to the incoming gas stream to effect primary filtering in the screen, and an outlet for the so-filtered gas stream.

3. A screening unit according to claim 2 wherein each baffle member is tubular having one open end for receiving the incoming gas stream and a closed end for directing the gas stream in the contraflow pattern.

- 15 -

0135390

4. A screening unit according to claim 3 wherein a plurality of tubular baffles of different diameter are disposed around a common axis and wherein the axial extent of at least one tubular baffle is different from the remaining tubular baffles.

5. A screening unit according to any preceding claim wherein the multiplicity of tortuous paths is formed by a plurality of superimposed gauzes made from a material which is inert relative to the liquid particles and the gas stream.

6. A screening unit according to any one of claims 1 to 4 including a plurality of bodies of regular or irregular geometric shape assembled so that interstitial spaces between adjacent bodies form the said multiplicity of tortuous paths.

7. A screening unit according to claim 6 wherein the bodies are of rounded form such as spheroids, solids of revolution having rounded ends and ellipsoids.

8. A screening unit according to claim 6 wherein the bodies are supported in at least one layer and are disposed between two surfaces which define a generally divergent or convergent chamber relative to the direction of the incoming particle laden gas stream.

- 16 -

0135390

9. A screening unit according to claim 5 wherein at least one of the said two surfaces is formed with a spiral channel for supporting the bodies.

10. A screening unit according to claim 8 or claim 9 wherein the convergent and divergent chambers converge or diverge with respect to their axes of symmetry within an angular range of 15° to 75° and, preferably, within the range of 25° to 50°.

11. A nebulizer including a screening unit according to any one of claims 1 to 9 and a nozzle assembly, the nozzle assembly comprising a gas nozzle and a spray nozzle for producing an aerosol spray which is directed towards the baffle system for primary filtration prior to secondary filtration in the screen.

FIGURE 1.

FIGURE 2.

3/6

SECTION X-X

FIGURE 3.

FIGURE 4.

FIGURE 5.

FIGURE 6.